(19) Europäisches Patentamt
European Patent Office
Office européen des brevets

(11) EP 4 209 216 A1

(12) **EUROPEAN PATENT APPLICATION**
published in accordance with Art. 153(4) EPC

(43) Date of publication:
**12.07.2023 Bulletin 2023/28**

(21) Application number: **21886203.5**

(22) Date of filing: **26.10.2021**

(51) International Patent Classification (IPC):
***A61K 31/4545*** *(2006.01)* ***A61K 39/395*** *(2006.01)*
***A61K 45/00*** *(2006.01)* ***A61P 15/00*** *(2006.01)*
***A61P 35/00*** *(2006.01)* ***A61P 35/04*** *(2006.01)*
***A61P 43/00*** *(2006.01)*

(52) Cooperative Patent Classification (CPC):
**A61K 31/4545; A61K 39/395; A61K 45/00; A61P 15/00; A61P 35/00; A61P 35/04; A61P 43/00**

(86) International application number:
**PCT/JP2021/039490**

(87) International publication number:
**WO 2022/092085 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.10.2020 JP 2020180455**

(71) Applicant: **Eisai R&D Management Co., Ltd**
**Bunkyo-ku, Tokyo 112-8088 (JP)**

(72) Inventors:
- **HOSHI, Taisuke**
  **Tsukuba-shi, Ibaraki 300-2635 (JP)**
- **KATO, Yu**
  **Tsukuba-shi, Ibaraki 300-2635 (JP)**
- **OZAWA, Yoichi**
  **Tsukuba-shi, Ibaraki 300-2635 (JP)**
- **KAWANO, Satoshi**
  **Tsukuba-shi, Ibaraki 300-2635 (JP)**
- **KODAMA, Kotaro**
  **Tsukuba-shi, Ibaraki 300-2635 (JP)**
- **MIYANO, Saori**
  **Tsukuba-shi, Ibaraki 300-2635 (JP)**

(74) Representative: **Hoffmann Eitle**
**Patent- und Rechtsanwälte PartmbB**
**Arabellastraße 30**
**81925 München (DE)**

# (54) PHARMACEUTICAL COMPOSITION FOR TREATING TUMORS

(57) Provided is a pharmaceutical composition for treating a tumor, comprising 5-((2-(4-(1-(2-hydroxyethyl)piperidin-4-yl)benzamide)pyridin-4-yl)oxy)-6-(2-methoxyethoxy)-N-methyl-1H-indole-1-carboxamide or its pharmaceutically acceptable salt, which is to be administered in combination with a PD-1 antagonist.

*Fig.1*

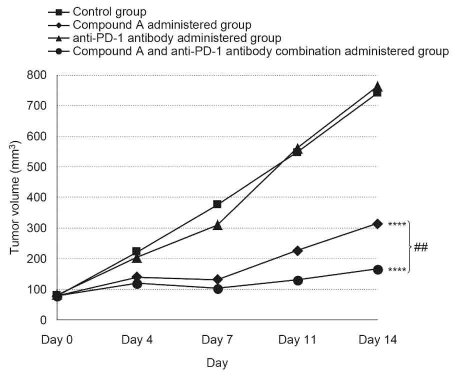


EP 4 209 216 A1

## Description

### Technical Field

The present invention relates to a pharmaceutical composition for treating tumor which combines the use of a monocyclic pyridine derivative or its pharmaceutically acceptable salt, which acts to inhibit fibroblast growth factor receptor (FGFR), with a PD-1 antagonist. More specifically, it relates to a pharmaceutical composition for treating tumor that comprises 5-((2-(4-(1-(2-hydroxyethyl)piperidin-4-yl)benzamide)pyridin-4-yl)oxy)-6-(2-methoxyethoxy)-N-methyl-1H-indole-1-carboxamide or its pharmaceutically acceptable salt, to be administered in combination with a PD-1 antagonist.

### Background Art

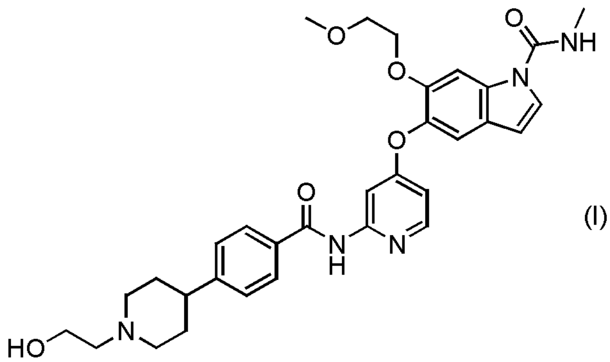

(I)

The compound 5-((2-(4-(1-(2-hydroxyethyl)piperidin-4-yl)benzamide)pyridin-4-yl)oxy)-6-(2-methoxyethoxy)-N-methyl-1H-indole-1-carboxamide, represented by formula (I), is known as an inhibitor against fibroblast growth factor receptors (FGFR) 1, 2 and 3, and has been reported to have a cell growth inhibitory effect against stomach cancer, lung cancer, bladder cancer and endometrial cancer (PTL 1). The compound has also been reported to have a high therapeutic effect for bile duct cancer (PTL 2), breast cancer (PTL 3) and hepatocellular carcinoma (PTL 4). Known pharmaceutically acceptable salts of the compound include succinic acid salts and maleic acid salts (PTL 5).

PD-L1 ligand and PD-L2 ligand that are expressed on tumor cells bind to PD-1 receptor expressed on activated T cells, thereby suppressing the activity of T cells. Anti-PD-1 antibody binds to PD-1 receptor to inhibit binding of PD-L1 ligand and PD-L2 ligand to PD-1 receptor, and thus performs a role in maintaining activation of T cells. The ability of T cells to attack tumor cells is thereby maintained. Drugs that carry out this function are known as immune checkpoint inhibitors, and are used as antitumor agents (NPL 1).

### Citation List

#### Patent Literature

[PTL 1] U.S. Patent Application Publication No. 2014-235614
[PTL 2] U.S. Patent Application Publication No. 2018-0015079
[PTL 3] U.S. Patent Application Publication No. 2018-0303817
[PTL 4] International Patent Publication No. WO2019/189241
[PTL 5] U.S. Patent Application Publication No. 2017-0217935

#### Non-Patent Literature

[NPL 1] The Journal of Clinical Investigation, Volume 125, Number 9, September 2015

## Summary of Invention

### Technical Problem

It is an object of the present invention to provide a pharmaceutical composition for treating tumor to be administered in combination with multiple different drugs.

### Solution to Problem

As a result of conducting much research in light of the current situation, the present inventors have completed this invention upon finding that the compound represented by formula (I) above exhibits a high therapeutic effect against tumors such as breast cancer when administered in combination with a PD-1 antagonist.

Specifically, the disclosure provides the following [1] to [93].

[1] A pharmaceutical composition for treating a tumor, comprising the compound represented by formula (I) or its pharmaceutically acceptable salt, which is to be administered in combination with a PD-1 antagonist.

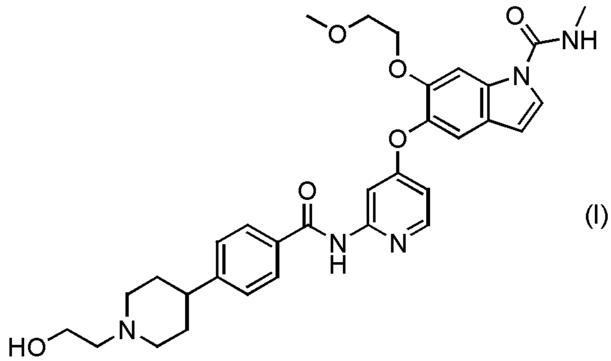

(I)

[2] A pharmaceutical composition for treating a tumor, comprising a PD-1 antagonist, which is to be administered in combination with the compound represented by formula (I) or its pharmaceutically acceptable salt.

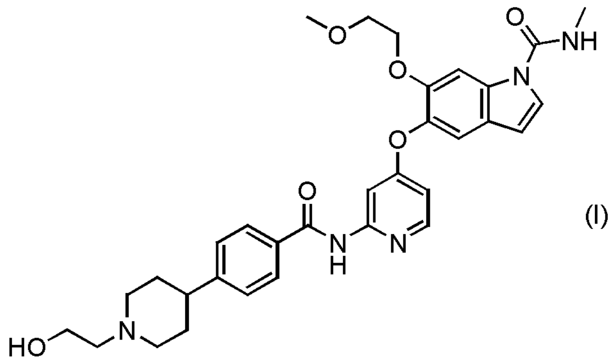

(I)

[3] The pharmaceutical composition according to [1] or [2], wherein the compound represented by formula (I) or its pharmaceutically acceptable salt and the PD-1 antagonist are each administered simultaneously, separately, continuously or at a time difference.

[4] The pharmaceutical composition according to any one of [1] to [3], wherein the pharmaceutically acceptable salt of the compound represented by formula (I) is a 1.5 succinate.

[5] The pharmaceutical composition according to [4], wherein the 1.5 succinate of the compound represented by formula (I) is administered at 1 mg to 500 mg per day.

[6] The pharmaceutical composition according to any one of [1] to [5], wherein the PD-1 antagonist is an anti-PD-1 antibody.

[7] The pharmaceutical composition according to [6], wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab,

Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

[8] The pharmaceutical composition according to [7], wherein the anti-PD-1 antibody is selected from the group consisting of Nivolumab, Pembrolizumab, Cemiplimab, Sintilimab and Toripalimab.

[9] The pharmaceutical composition according to [8], wherein the anti-PD-1 antibody is Nivolumab.

[10] The pharmaceutical composition according to [9], wherein the Nivolumab is administered at 3 mg/kg (body weight) per dose at 2-week intervals, 240 mg per dose at 2-week intervals, 360 mg per dose at 3-week intervals or 480 mg per dose at 4-week intervals.

[11] The pharmaceutical composition according to [8], wherein the anti-PD-1 antibody is Pembrolizumab.

[12] The pharmaceutical composition according to [11], wherein the Pembrolizumab is administered at 200 mg per dose at 3-week intervals or 400 mg per dose at 6-week intervals.

[13] The pharmaceutical composition according to any one of [1] to [12], wherein the tumor is breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin's lymphoma.

[14] The pharmaceutical composition according to any one of [1] to [12], wherein the tumor is breast cancer, stomach cancer, non-small-cell lung cancer, bladder cancer, endometrial cancer, hepatocellular carcinoma or bile duct cancer.

[15] The pharmaceutical composition according to any one of [1] to [12], wherein the tumor is breast cancer.

[16] The pharmaceutical composition according to any one of [13] to [15], wherein the breast cancer is locally advanced breast cancer, metastatic breast cancer or recurrent breast cancer.

[17] The pharmaceutical composition according to any one of [13] to [16], wherein the breast cancer expresses fibroblast growth factor receptor (FGFR).

[18] The pharmaceutical composition according to [17], wherein the FGFR is FGFR1, FGFR2 or FGFR3.

[19] A therapeutic agent for a tumor, comprising the compound represented by formula (I) or its pharmaceutically acceptable salt, which is to be administered in combination with a PD-1 antagonist.

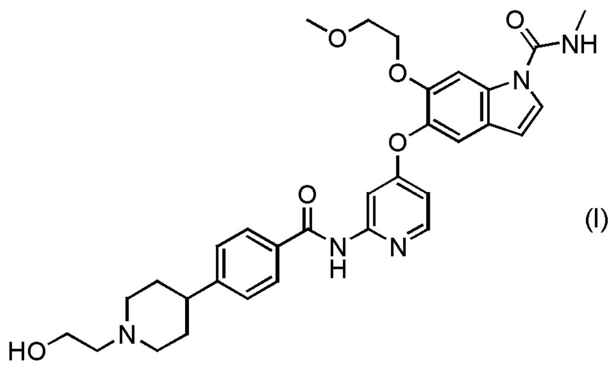

(I)

[20] A therapeutic agent for a tumor, comprising a PD-1 antagonist, which is to be administered in combination with the compound represented by formula (I) or its pharmaceutically acceptable salt.

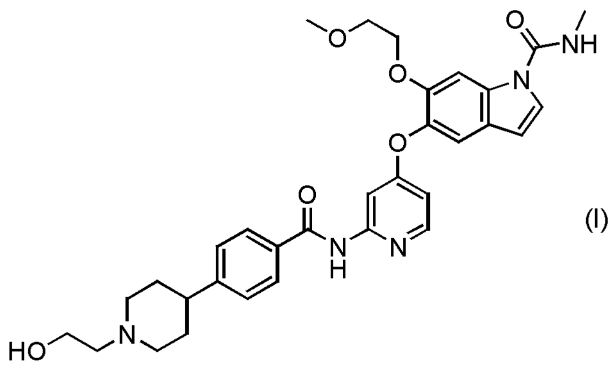

(I)

[21] The therapeutic agent for a tumor according to [19] or [20], wherein the compound represented by formula (I) or its pharmaceutically acceptable salt and the PD-1 antagonist are each administered simultaneously, separately,

continuously or at a time difference.

[22] The therapeutic agent for a tumor according to any one of [19] to [21], wherein the pharmaceutically acceptable salt of the compound represented by formula (I) is a 1.5 succinate.

[23] The therapeutic agent for a tumor according to [22], wherein the 1.5 succinate of the compound represented by formula (I) is administered at 1 mg to 500 mg per day.

[24] The therapeutic agent for a tumor according to any one of [19] to [23], wherein the PD-1 antagonist is an anti-PD-1 antibody.

[25] The therapeutic agent for a tumor according to [24], wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

[26] The therapeutic agent for a tumor t according to [25], wherein the anti-PD-1 antibody is selected from the group consisting of Nivolumab, Pembrolizumab, Cemiplimab, Sintilimab and Toripalimab.

[27] The therapeutic agent for a tumor according to [26], wherein the anti-PD-1 antibody is Nivolumab.

[28] The therapeutic agent for a tumor according to [27], wherein the Nivolumab is administered at 3 mg/kg (body weight) per dose at 2-week intervals, 240 mg per dose at 2-week intervals, 360 mg per dose at 3-week intervals or 480 mg per dose at 4-week intervals.

[29] The therapeutic agent for a tumor according to [26], wherein the anti-PD-1 antibody is Pembrolizumab.

[30] The therapeutic agent for a tumor according to [29], wherein the Pembrolizumab is administered at 200 mg per dose at 3-week intervals or 400 mg per dose at 6-week intervals.

[31] The therapeutic agent for a tumor according to any one of [19] to [30], wherein the tumor is breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin’s lymphoma.

[32] The therapeutic agent for a tumor according to any one of [19] to [30], wherein the tumor is breast cancer, stomach cancer, non-small-cell lung cancer, bladder cancer, endometrial cancer, hepatocellular carcinoma or bile duct cancer.

[33] The therapeutic agent for a tumor according to any one of [19] to [30], wherein the tumor is breast cancer.

[34] The therapeutic agent for a tumor according to any one of [31] to [33], wherein the breast cancer is locally advanced breast cancer, metastatic breast cancer or recurrent breast cancer.

[35] The therapeutic agent for a tumor according to any one of [31] to [34], wherein the breast cancer expresses fibroblast growth factor receptor (FGFR).

[36] The therapeutic agent for a tumor according to [35], wherein the FGFR is FGFR1, FGFR2 or FGFR3.

[37] A method for treating a tumor, which includes administering a pharmaceutically acceptable salt of the compound represented by formula (I) and a PD-1 antagonist to a patient in need thereof.

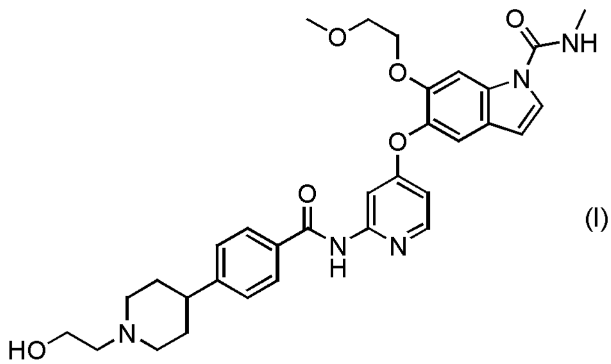

(I)

[38] The method according to [37], wherein the pharmaceutically acceptable salt of the compound represented by formula (I) and the PD-1 antagonist are each administered simultaneously, separately, continuously or at a time difference.

[39] The method according to [37] or [38], wherein the pharmaceutically acceptable salt of the compound represented by formula (I) is a 1.5 succinate.

[40] The method according to [39], wherein the 1.5 succinate of the compound represented by formula (I) is administered at 1 mg to 500 mg per day.

[41] The method according to any one of [37] to [40], wherein the PD-1 antagonist is an anti-PD-1 antibody.

[42] The method according to [41], wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

[43] The method according to [42], wherein the anti-PD-1 antibody is selected from the group consisting of Nivolumab, Pembrolizumab, Cemiplimab, Sintilimab and Toripalimab.

[44] The method according to [43], wherein the anti-PD-1 antibody is Nivolumab.

[45] The method according to [44], wherein the Nivolumab is administered at 3 mg/kg (body weight) per dose at 2-week intervals, 240 mg per dose at 2-week intervals, 360 mg per dose at 3-week intervals or 480 mg per dose at 4-week intervals.

[46] The method according to [43], wherein the anti-PD-1 antibody is Pembrolizumab.

[47] The method according to [46], wherein the Pembrolizumab is administered at 200 mg per dose at 3-week intervals or 400 mg per dose at 6-week intervals.

[48] The method according to any one of [37] to [47], wherein the tumor is breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin’s lymphoma.

[49] The method according to any one of [37] to [47], wherein the tumor is breast cancer, stomach cancer, non-small-cell lung cancer, bladder cancer, endometrial cancer, hepatocellular carcinoma or bile duct cancer.

[50] The method according to any one of [37] to [47], wherein the tumor is breast cancer.

[51] The method according to any one of [48] to [50], wherein the breast cancer is locally advanced breast cancer, metastatic breast cancer or recurrent breast cancer.

[52] The method according to any one of [48] to [51], wherein the breast cancer expresses fibroblast growth factor receptor (FGFR).

[53] The method according to [52], wherein the FGFR is FGFR1, FGFR2 or FGFR3.

[54] The use of the compound represented by formula (I) or its pharmaceutically acceptable salt, in the manufacture of a pharmaceutical composition for treating a tumor which is to be administered in combination with a PD-1 antagonist.

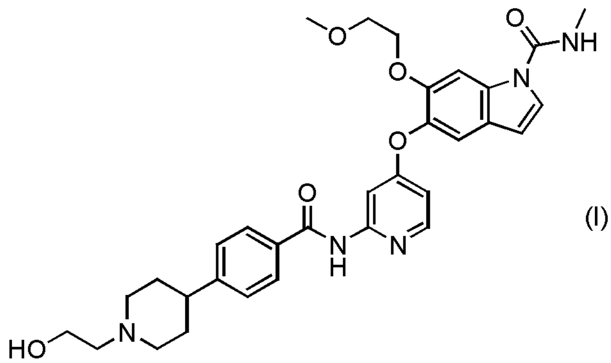

(I)

[55] The use of a PD-1 antagonist in the manufacture of a pharmaceutical composition for tumor treatment which is to be administered in combination with the compound represented by formula (I) or its pharmaceutically acceptable salt.

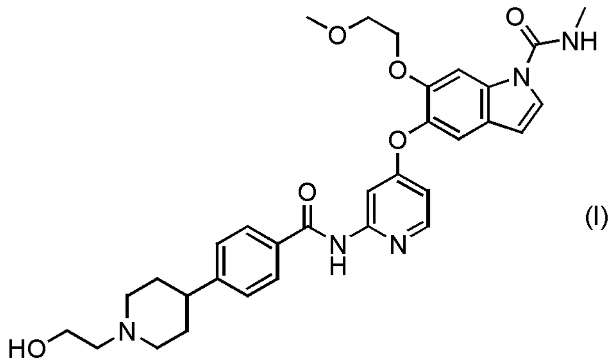

(I)

[56] The use according to [54] or [55], wherein the compound represented by formula (I) or its pharmaceutically acceptable salt and the PD-1 antagonist are each administered simultaneously, separately, continuously or at a time difference.

[57] The use according to any one of [54] to [56], wherein the pharmaceutically acceptable salt of the compound represented by formula (I) is a 1.5 succinate.

[58] The use according to [57], wherein the 1.5 succinate of the compound represented by formula (I) is administered at 1 mg to 500 mg per day.

[59] The use according to any one of [54] to [58], wherein the PD-1 antagonist is an anti-PD-1 antibody.

[60] The use according to [59], wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

[61] The use according to [60], wherein the anti-PD-1 antibody is selected from the group consisting of Nivolumab, Pembrolizumab, Cemiplimab, Sintilimab and Toripalimab.

[62] The use according to [61], wherein the anti-PD-1 antibody is Nivolumab.

[63] The use according to [62], wherein the Nivolumab is administered at 3 mg/kg (body weight) per dose at 2-week intervals, 240 mg per dose at 2-week intervals, 360 mg per dose at 3-week intervals or 480 mg per dose at 4-week intervals.

[64] The use according to [61], wherein the anti-PD-1 antibody is Pembrolizumab.

[65] The use according to [64], wherein the Pembrolizumab is administered at 200 mg per dose at 3-week intervals or 400 mg per dose at 6-week intervals.

[66] The use according to any one of [54] to [65], wherein the tumor is breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin’s lymphoma.

[67] The use according to any one of [54] to [66], wherein the tumor is breast cancer, stomach cancer, non-small-cell lung cancer, bladder cancer, endometrial cancer, hepatocellular carcinoma or bile duct cancer.

[68] The use according to any one of [54] to [67], wherein the tumor is breast cancer.

[69] The use according to any one of [66] to [68], wherein the breast cancer is locally advanced breast cancer, metastatic breast cancer or recurrent breast cancer.

[70] The use according to any one of [66] to [69], wherein the breast cancer expresses fibroblast growth factor receptor (FGFR).

[71] The use according to [70], wherein the FGFR is FGFR1, FGFR2 or FGFR3.

[72] The compound represented by formula (I) or its pharmaceutically acceptable salt for use in treating a tumor, which is to be administered in combination with a PD-1 antagonist.

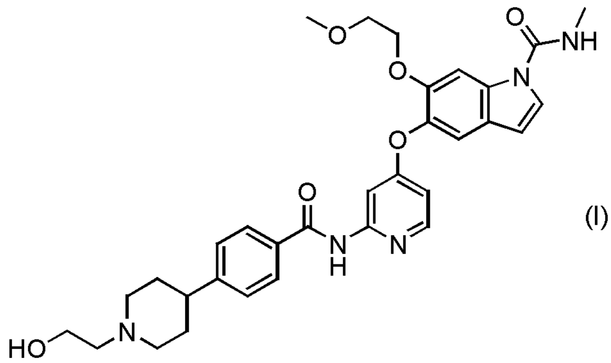

(I)

[73] A PD-1 antagonist for use in treating a tumor, which is to be administered in combination with the compound represented by formula (I) or its pharmaceutically acceptable salt.

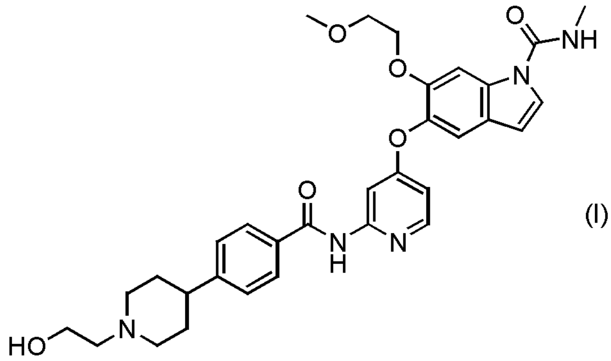

(I)

[74] The compound represented by formula (I) or its pharmaceutically acceptable salt or the PD-1 antagonist according to [72] or [73], wherein the compound represented by formula (I) or its pharmaceutically acceptable salt and the PD-1 antagonist are each administered simultaneously, separately, continuously or at a time difference.
[75] The compound represented by formula (I) or its pharmaceutically acceptable salt or the PD-1 antagonist according to any one of [72] to [74], wherein the pharmaceutically acceptable salt of the compound represented by formula (I) is a 1.5 succinate.
[76] The compound represented by formula (I) or its pharmaceutically acceptable salt or the PD-1 antagonist according to [75], wherein the 1.5 succinate of the compound represented by formula (I) is administered at 1 mg to 500 mg per day.
[77] The compound represented by formula (I) or its pharmaceutically acceptable salt or the PD-1 antagonist according to any one of [72] to [76], wherein the PD-1 antagonist is an anti-PD-1 antibody.
[78] The compound represented by formula (I) or its pharmaceutically acceptable salt or the PD-1 antagonist according to [77], wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.
[79] The compound represented by formula (I) or its pharmaceutically acceptable salt or the PD-1 antagonist according to [78], wherein the anti-PD-1 antibody is selected from the group consisting of Nivolumab, Pembrolizumab, Cemiplimab, Sintilimab and Toripalimab.
[80] The compound represented by formula (I) or its pharmaceutically acceptable salt or the PD-1 antagonist according to [79], wherein the anti-PD-1 antibody is Nivolumab.
[81] The compound represented by formula (I) or its pharmaceutically acceptable salt or the PD-1 antagonist according to [80], wherein the Nivolumab is administered at 3 mg/kg (body weight) per dose at 2-week intervals, 240 mg per dose at 2-week intervals, 360 mg per dose at 3-week intervals or 480 mg per dose at 4-week intervals.
[82] The compound represented by formula (I) or its pharmaceutically acceptable salt or the PD-1 antagonist according to [79], wherein the anti-PD-1 antibody is Pembrolizumab.
[83] The compound represented by formula (I) or its pharmaceutically acceptable salt or the PD-1 antagonist according to [82], wherein the Pembrolizumab is administered at 200 mg per dose at 3-week intervals or 400 mg per

dose at 6-week intervals.

[84] The compound represented by formula (I) or its pharmaceutically acceptable salt or the PD-1 antagonist according to any one of [72] to [83], wherein the tumor is breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin's lymphoma.

[85] Compound represented by formula (I) or its pharmaceutically acceptable salt or the PD-1 antagonist according to any one of [72] to [84], wherein the tumor is breast cancer, stomach cancer, non-small-cell lung cancer, bladder cancer, endometrial cancer, hepatocellular carcinoma or bile duct cancer.

[86] The compound represented by formula (I) or its pharmaceutically acceptable salt or the PD-1 antagonist according to any one of [72] to [85], wherein the tumor is breast cancer.

[87] The compound represented by formula (I) or its pharmaceutically acceptable salt or the PD-1 antagonist according to any one of [84] to [86], wherein the breast cancer is locally advanced breast cancer, metastatic breast cancer or recurrent breast cancer.

[88] The compound represented by formula (I) or its pharmaceutically acceptable salt or the PD-1 antagonist according to any one of [84] to [87], wherein the breast cancer expresses fibroblast growth factor receptor (FGFR).

[89] The compound represented by formula (I) or its pharmaceutically acceptable salt or the PD-1 antagonist according to [88], wherein the FGFR is FGFR1, FGFR2 or FGFR3.

[90] A kit for treating a tumor which is provided with a formulation comprising the compound represented by formula (I) or its pharmaceutically acceptable salt, and a formulation comprising a PD-1 antagonist.

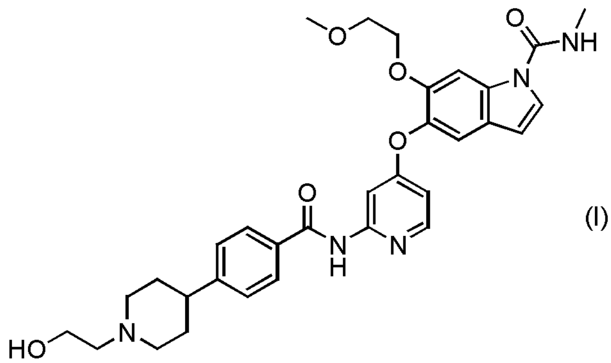

(I)

[91] The kit according to [90], wherein the compound represented by formula (I) or its pharmaceutically acceptable salt and the PD-1 antagonist are each administered simultaneously, separately, continuously or at a time difference.

[92] A combination for treating a tumor that includes the compound represented by formula (I) or its pharmaceutically acceptable salt, and a PD-1 antagonist.

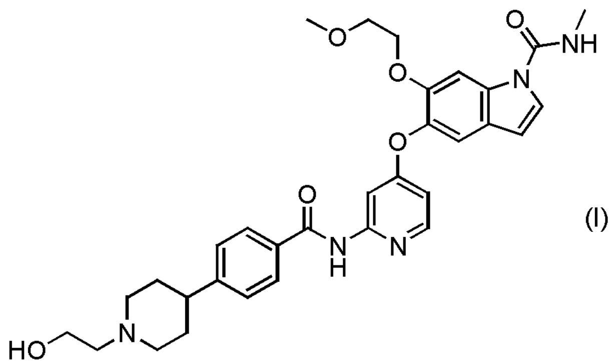

(I)

[93] The combination according to [92], wherein the compound represented by formula (I) or its pharmaceutically acceptable salt and the PD-1 antagonist are each administered simultaneously, separately, continuously or at a time difference.

## Advantageous Effects of Invention

By administering a combination of the compound represented by formula (I) and a PD-1 antagonist it is possible

to exhibit an effect of reducing tumor volume for tumors such as breast cancer.

## Brief Description of Drawings

Fig. 1 is a graph showing transition of mean tumor volume of different groups after initiating drug administration, for Example 1.
Fig. 2 is a graph showing transition of mean tumor volume of different groups after initiating drug administration, for Example 2.
Fig. 3 is a graph showing transition of mean tumor volume of different groups after initiating drug administration, for Example 3.

## Description of Embodiments

The compound represented by formula (I) and its pharmaceutically acceptable salts according to the disclosure can be produced by the methods described in PTL 1.

As used herein, "pharmaceutically acceptable salt" refers to a salt of an inorganic acid, a salt of an organic acid, or a salt of an acidic amino acid, for example, which are pharmaceutically acceptable salts. Solvates of pharmaceutically acceptable salts of the compound of the disclosure, such as anhydrides and hydrates of the pharmaceutically acceptable salts, are also included.

Examples of salts with inorganic acids include salts with hydrochloric acid, hydrobromic acid, sulfuric acid, nitric acid and phosphoric acid.

Examples of salts with organic acids include salts with acetic acid, succinic acid, fumaric acid, maleic acid, tartaric acid, citric acid, lactic acid, stearic acid, benzoic acid, methanesulfonic acid, ethanesulfonic acid and p-toluenesulfonic acid.

Examples of salts with acidic amino acids include salts with aspartic acid and glutamic acid.

According to one embodiment, the pharmaceutically acceptable salt is a succinic acid salt or maleic acid salt. According to another embodiment, the pharmaceutically acceptable salt is a succinic acid salt. According to yet another embodiment, the pharmaceutically acceptable salt is a 1.5 succinate (hereunder, the 1.5 succinate of the compound represented by formula (I) will be referred to as "compound A").

The compound represented by formula (I) or its pharmaceutically acceptable salt according to the disclosure may be administered by injection (intravenous injection, intraarterial injection or local injection), or by an oral, intranasal, transdermal or transpulmonary route, or by eye drop, and for example, injection may be intravenous injection, subcutaneous injection, intradermal injection or intraarterial injection, or local injection into the target cells or organ. The dosage form of the compound represented by formula (I) or its pharmaceutically acceptable salt for oral administration may be a tablet, powder, granules, syrup, capsule or internal liquid drug, for example. The dosage form of the compound represented by formula (I) or its pharmaceutically acceptable salt for parenteral administration may also be injection, drip, eye drop, ointment, suppository, suspension, poultice, lotion, aerosol or plaster, or it may be injection or drip, according to one embodiment. The compound represented by formula (I) or its pharmaceutically acceptable salt according to the disclosure can be formulated by a method described in Japanese Pharmacopoeia, 17th Edition (JP), U.S. Pharmacopeia (USP) or European Pharmacopeia (EP), for example.

The dose of the compound represented by formula (I) or pharmaceutically acceptable salt may be appropriately selected according to the severity of symptoms, the age, gender, body weight and sensitivity of the patient, the method of administration, the period of administration, the interval of administration and the type of medical formulation. The number of doses and frequency of administration of the pharmaceutical composition of the disclosure may be determined by a person skilled in the art as an appropriate and suitable number of doses and frequency of administration for the conditions under which the immune checkpoint inhibitor is to be administered (the dose interval, number of doses and administration period). For oral administration to an adult (60 kg body weight), the dose will generally be 1 mg to 500 mg per day, being 10 mg to 300 mg according to one embodiment, or 20 mg to 200 mg according to another embodiment. It may be administered as 1 to 3 dosages per day.

The PD-1 antagonist of the disclosure may include any compound or biomolecule that blocks binding of PD-L1 expressed by cancer cells to PD-1 expressed on immunocytes (T cells, B cells or Natural Killer T (NKT) cells), or that blocks binding of PD-L2 expressed by cancer cells to PD-1 expressed on immunocytes. The PD-1 antagonist blocks binding of human PD-L1 to human PD-1, and according to one embodiment it blocks binding of both human PD-L1 and PD-L2 to human PD-1. The amino acid sequence of human PD-1 can be confirmed at NCBI Locus No.: NP_005009. The amino acid sequences of human PD-L1 and PD-L2 can be confirmed as NCBI Locus No.: NP_054862 and NP_079515, respectively.

The PD-1 antagonist of the disclosure may also include a monoclonal antibody (mAb) that specifically binds to PD-1 or PD-L1, or specifically binds to human PD-1 or human PD-L1, or its antigen-binding fragment. The mAb may be a human antibody, humanized antibody or chimeric antibody, and it may also include the human constant region. The human constant region is selected from the group consisting of the IgG1, IgG2, IgG3 and IgG4 constant regions, and according to one embodiment the human constant region is the IgG1 or IgG4 constant region. The antigen-binding fragment may be selected from the group consisting of Fab, Fab'-SH, $F(ab')_2$, scFv and Fv fragments.

An example of a PD-1 antagonist is anti-PD-1 antibody, and according to one embodiment it is anti-human PD-1 antibody, while according to a more specific embodiment it is anti-human PD-1 monoclonal antibody (anti-human PD-1 mAb). Examples of mAb that bind to human PD-1 are described in US Patent No. 7488802, US Patent No. 7521051, US Patent No. 8008449, US Patent No. 8354509, US Patent No. 8168757, International Patent Publication No. WO2004/004771, International Patent Publication No. WO2004/072286, International Patent Publication No. WO2004/056875 and U.S. Patent Application Publication No. 2011/0271358. When the PD-1 antagonist is an anti-human PD-1 monoclonal antibody, "anti-human PD-1 monoclonal antibody" includes Nivolumab, Pembrolizumab, Cemiplimab, Sintilimab and Toripalimab. When the PD-1 antagonist is an anti-PD-1 antibody, "anti-PD-1 antibody" further includes Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

Another example of a PD-1 antagonist is anti-PD-L1 antibody, and according to one embodiment it is anti-human PD-L1 antibody, while according to a more specific embodiment it is anti-human PD-L1 monoclonal antibody (anti-human PD-L1 mAb). When the PD-1 antagonist is an anti-PD-L1 antibody, "anti-PD-L1 antibody" includes Atezolizumab, Avelumab, Durvalumab, Manelimab, Pacmilimab, Envafolimab, Cosibelimab, BMS-936559, STI-1014, KN035, LY33,00054, HLX20, SHR-1316, CS1001, MSB2311, BGB-A333 and KL-A16.

The PD-1 antagonist of the disclosure may be administered by injection (intravenous injection, intraarterial injection or local injection), or by an oral, intranasal, transdermal or transpulmonary route, or by eye drop, and for example, injection may be intravenous injection, subcutaneous injection, intradermal injection or intraarterial injection, or local injection into the target cells or organ. The dosage form of a formulation comprising the PD-1 antagonist for oral administration may be a tablet, powder, granules, syrup, capsule or internal liquid drug, for example. The dosage form of a formulation comprising the PD-1 antagonist for parenteral administration may be injection, drip, eye drop, ointment, suppository, suspension, poultice, lotion, aerosol or plaster, or it may be injection or drip, according to one embodiment. The PD-1 antagonist of the disclosure can be formulated by a method described in Japanese Pharmacopoeia, 17th Edition (JP), U.S. Pharmacopeia (USP) or European Pharmacopeia (EP), for example.

When the PD-1 antagonist is an anti-PD-1 antibody, the anti-PD-1 antibody may be provided as a liquid drug, or it may be prepared as a liquid solution of freeze-dried powder in sterile water for injection before use.

When an anti-human PD-1 mAb, as a PD-1 antagonist, is to be administered as a single agent to a patient, the dose will differ significantly depending on the type of disease being treated, and the age, gender, body weight and severity of symptoms of the patient. The anti-human PD-1 mAb is administered in a dose of 1, 2, 3, 5 or 10 mg/kg (body weight), at intervals of about 14 days ($\pm$2 days), about 21 days ($\pm$2 days) or about 30 days ($\pm$2 days).

When Pembrolizumab is administered as a PD-1 antagonist, it may be intravenously administered in a manner and dosage selected from among 1 mg/kg (body weight) at 2-week intervals, 2 mg/kg (body weight) at 2-week intervals, 3 mg/kg (body weight) at 2-week intervals, 5 mg/kg (body weight) at 2-week intervals, 10 mg (body weight) at 2-week intervals, 1 mg/kg (body weight) at 3-week intervals, 2 mg/kg (body weight) at 3-week intervals, 3 mg/kg (body weight) at 3-week intervals, 5 mg/kg (body weight) at 3-week intervals and 10 mg/kg (body weight) at 3-week intervals. According to another aspect, Pembrolizumab may be intravenously administered at 200 mg per dose at 3-week intervals or 400 mg per dose at 6-week intervals. Pembrolizumab may be administered, for example, as an intravenous infusion of a liquid drug containing Pembrolizumab, L-histidine, L-histidine hydrochloride hydrate, refined sucrose, and polysorbate 80, over a period of about 30 minutes.

When Nivolumab is administered as a PD-1 antagonist, it may be intravenously administered in a manner and dosage selected from among 1 mg/kg (body weight) at 2-week intervals, 2 mg/kg (body weight) at 2-week intervals, 3 mg/kg (body weight) at 2-week intervals, 4 mg/kg (body weight) at 2-week intervals, 5 mg/kg (body weight) at 2-week intervals, 6 mg/kg (body weight) at 2-week intervals, 1 mg/kg (body weight) at 3-week intervals, 2 mg/kg (body weight) at 3-week intervals, 3 mg/kg (body weight) at 3-week intervals, 4 mg/kg (body weight) at 3-week intervals, 5 mg/kg (body weight) at 3-week intervals, 6 mg/kg (body weight) at 3-week intervals, 8 mg/kg (body weight) at 3-week intervals and 10 mg/kg (body weight) at 3-week intervals. According to another aspect, Nivolumab may be intravenously administered at 240 mg per dose at 2-week intervals, 360 mg per dose at 3-week intervals or 480 mg per dose at 4-week intervals. Nivolumab may be administered, for example as an intravenous infusion of a liquid drug containing Nivolumab, D-mannitol, sodium citrate hydrate, sodium chloride, diethylenetriaminepentaacetic acid, polysorbate 80 and a pH regulator, over a period of 30 minutes or longer.

When Cemiplimab is administered as the PD-1 antagonist, it may be intravenously administered, for example, at 350 mg per dose at 3-week intervals.

The pharmaceutical composition for treating a tumor of the disclosure may be orally administered in the form of a solid formulation such as a tablet, granules, fine granules, powder or capsule, or a liquid drug, jelly or syrup. The pharmaceutical composition for treating a tumor of the disclosure may also be parenterally administered in the form of an injection, suppository, ointment or poultice. The pharmaceutical composition for treating a tumor of the disclosure can be formulated by a method described in Japanese Pharmacopoeia, 17th Edition (JP), U.S. Pharmacopeia (USP) or European Pharmacopeia (EP).

The form of administering the compound represented by formula (I) or its pharmaceutically acceptable salt and the PD-1 antagonist of the disclosure is not particularly restricted, as it is sufficient if the compound represented by formula (I) or its pharmaceutically acceptable salt and the PD-1 antagonist are administered in combination. For example, the compound represented by formula (I) or its pharmaceutically acceptable salt and the PD-1 antagonist may each be administered to a patient simultaneously, separately, continuously or at a time difference. The term "simultaneously" may mean that each component is administered within the same time period or exactly at the same time, or via the same route of administration. The term may also mean that both components are administered without a notable interval so that they can exhibit an additive effect, and preferably a synergistic effect. The term "separately" means that the components are administered at different intervals or at different frequencies, or by different routes of administration. The term "continuously" means that the components are administered during a fixed period by either the same route or different routes of administration, in any order. The phrase "at a time difference" means that the components are administered over different intervals for the respective components, by either the same route or different routes of administration. When the PD-1 antagonist is administered during a period of one cycle of administration of the compound represented by formula (I) or its pharmaceutically acceptable salt, or during a period of repeated cycles, this is considered to be administration of both in combination.

There is no particular restriction on the manner of combination of the compound represented by formula (I) or its pharmaceutically acceptable salt with the PD-1 antagonist, and any method known to those skilled in the art may be used.

The type of tumor to be treated by the pharmaceutical composition for treating a tumor of the disclosure is not particularly restricted, and may be breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin's lymphoma, for example. According to one embodiment, the tumor to be treated is breast cancer, stomach cancer, non-small-cell lung cancer, bladder cancer, endometrial cancer, hepatocellular carcinoma or bile duct cancer. According to another embodiment, the tumor to be treated is breast cancer.

Non-small-cell lung cancer, for the purpose of the present specification, includes lung squamous cell carcinoma. Bile duct cancer, for the purpose of the present specification, includes intrahepatic cholangiocarcinoma, extrahepatic cholangiocarcinoma, cystic duct cancer, gallbladder cancer and duodenal papilla cancer. Hepatocellular carcinoma, for the purpose of the present specification, means a benign or malignant tumor developing in liver cells. Breast cancer, for the purpose of the present specification, means a benign or malignant tumor developing in the mammary glands (lactiferous ducts or lobules). Included in this definition are locally advanced breast cancer, metastatic breast cancer, recurrent breast cancer and unresectable breast cancer.

## Examples

The present invention will now be explained in greater detail by the following examples. However, various other embodiments of the present invention may be implemented, and the invention is not to be interpreted as being limited to the Examples described herein.

### Example 1 Antitumor effect by combination of compound A and anti-PD-1 antibody in mouse breast cancer cell line (4T1-Luc2 AcGFP) graft model

The antitumor effect of administering compound A and anti-PD-1 antibody was evaluated in BALB/c mice (BALB/cAnNCrlCrlj, female, Japan Charles River) with 6 mice per group.

<Establishing mouse breast cancer cell line 4T1-Luc2 AcGFP>

The mouse breast cancer cell line 4T1 (ATCC) was seeded in a 6-well microplate (Falcon), at a cell count of $1.5 \times 10^5$ per well. The culture medium used was RPMI-1640 medium containing 10% FBS, penicillin, streptomycin and 2-mercaptoethanol (FujiFilm-Wako). The seeded cells were cultured overnight using an incubator under conditions of

5% $CO_2$, 37°C. The culture medium was replaced on the following day.

Vector pPBcmvIP-mcs was constructed from PB-CMV-MCS-EF1α-Puro vector (SBI; Catalog No.: PB510B-1), removing EF1-Puro and substituting IRES-Puro. A Luc2/AcGFP fusion gene was then inserted between the pPBcmvIP-mcs CMV promoter and IRES to express Luciferase (Luc) and Green Fluorescent Protein (GFP), to obtain vector pPBcmvIP Luc2-AcGFP. During the process of constructing each vector, insertion of the separated fragments was confirmed by an established method.

The vector pPBcmvIP Luc2-AcGFP in 10 mM Tris-Cl (pH 8.0) and 1 mM EDTA (pH 8.0) ("TE Buffer") (1.67 μg), Super PiggyBac Transposase Expression Vector (System Biosciences, LLC) in TE Buffer (0.33 μg) and X-tream-Gene reagent (Roche) (8 μL) were mixed, and the mixture was adjusted to a total amount of 210 μL with Opti-MEM (Thermo Fischer Scientific). The obtained mixture was allowed to stand at room temperature for 15 minutes, and then added to the cultured 4T1 cells. The medium was exchanged and subculturing was carried out in a T75 flask, after which culturing was continued under conditions of 5% $CO_2$, 37°C. After 4 days, culturing was carried out in 2 μg/mL puromycin-containing medium.

Cells were selected twice with a flow cytometer, using AcGFP positivity as the marker. A mycoplasma removal reagent was added to the cells and culturing was continued for 8 days.

Cells of the mouse breast cancer cell line 4T1-Luc2 AcGFP established by the method described above were suspended in HBSS (FujiFilm-Wako) to a concentration of $2 \times 10^7$/mL. The mixture was grafted into mammary gland fat of BALB/c (BALB/cAnNCrlCrlj, female, Japan Charles River) in amounts of 0.05 mL each, and the antitumor effect was evaluated.

On the 4th day after grafting, the long and short diameters of the tumors were measured using an electronic digital caliper (Digimatic™ caliper by Mitsutoyo Corp.). The mice were divided into groups in such a manner that the average tumor volumes were the same in each group. The tumor volumes were calculated by the following formula.

$$\text{Tumor volume (mm)} = \text{Long diameter (mm)} \times \text{short diameter (mm)} \times \text{short diameter (mm)}/2$$

Compound A was dissolved in water for injection (Otsuka Pharmaceutical Factory, Inc.) to a concentration of 2.5 mg/mL. The anti-mouse PD-1 antibody (BioXcell) was diluted with PBS(-) (FujiFilm-Wako) to a concentration of 1 mg/mL.

Mice in the compound A administration group were given compound A by oral administration at a dose of 25 mg/kg (10 mL/kg), once per day for 14 days. Mice in the anti-PD-1 antibody administration group were intraperitoneally administered a dose of 0.2 mg/mouse on day 0, day 4, day 7 and day 11, where the starting day of administration was defined as day 0. The mice in the compound A and anti-PD-1 antibody combination group were administered both drugs in the same manner described above.

With the starting day of administration defined as day 0, the tumor volume of each mouse was measured on day 4, day 7, day 11 and day 14. The mean tumor volumes in each group are shown in Table 1. Fig. 1 shows the mean measurement results for tumor volume. The tumor volume on day 14 was statistically analyzed by a One-way ANOVA statistical test, or if no significant result was found in the test, by the Tukey-Kramer method. The final significance level was as follows.

Comparison with control group ****:p <0.0001

Comparison with compound A administration group ##:p <0.01

As a result, a markedly superior antitumor effect was seen in the compound A and anti-PD-1 antibody combination group compared to the control group and the groups administered each agent alone.

[Table 1]

| | Day 0 | Day 4 | Day 7 | Day 11 | Day 14 |
|---|---|---|---|---|---|
| Control group ($mm^3$) | 78.6 | 221.7 | 375.1 | 546.8 | 741.7 |
| Compound A administered group ($mm^3$) | 77.3 | 139.0 | 131.2 | 226.0 | 314.6 |
| Anti-mouse PD-1 antibody administered group ($mm^3$) | 82.1 | 203.7 | 310.3 | 560.9 | 763.6 |

(continued)

| | Day 0 | Day 4 | Day 7 | Day 11 | Day 14 |
|---|---|---|---|---|---|
| Compound A and anti-mouse PD-1 antibody combination administered group ($mm^3$) | 78.1 | 118.3 | 102.5 | 130.0 | 165.9 |

Example 2 Antitumor effect by combination of compound A and anti-PD-1 antibody in mouse renal cell carcinoma cell line (RAG) graft model

The antitumor effect of administering compound A and anti-PD-1 antibody was evaluated in BALB/c mice (BALB/cAnNCrlCrlj, female, Japan Charles River) with 10 mice per group.

<Establishing mouse renal cell carcinoma cell line RAG>

The grafted cells were pre-conditioned in BALB/c mice. The mouse renal cell carcinoma cell line RAG (ATCC) was suspended in HBSS to a concentration of $2 \times 10^8$/mL. An equal volume of Matrigel™ matrix (Japan Becton Dickinson) was added to and thoroughly mixed with the suspension. The mixture was subcutaneously grafted on the right side of BALB/c mice (BALB/cAnNCrlCrlj, female, Japan Charles River), at 0.1 mL each. After grafting, the formed tumors were extracted and finely cut. A Tumor Dissociation Kit, Mouse (Miltenyi Biotec K.K.) was then used for stirring in a GentleMACS (Miltenyi Biotec K.K.) to obtain a monocellular suspension. After passing through a 70 μm cell strainer, the cells were collected by centrifugation and cultured in medium containing 10% bovine serum.

The mouse renal cell carcinoma cell line RAG established by this method was suspended in HBSS to a concentration of $2.5 \times 10^7$/mL. The mixture was subsequently grafted onto the right side of BALB/c (BALB/cAnNCrlCrlj, female, Japan Charles River) in amounts of 0.1 mL each, and the antitumor effect was evaluated.

On the 7th day after grafting, the long and short diameters of the tumors were measured using an electronic digital caliper (Digimatic™ caliper by Mitsutoyo Corp.). The mice were divided into groups in such a manner that the average tumor volumes were the same in each group. The tumor volumes were calculated by the following formula.

$$\text{Tumor volume (mm)} = \text{Long diameter (mm)} \times \text{short diameter (mm)} \times \text{short diameter (mm)}/2$$

Compound A was dissolved in water for injection (Otsuka Pharmaceutical Factory, Inc.) to a concentration of 2.5 mg/mL. The anti-mouse PD-1 antibody (BioXcell) was diluted with physiological saline (Otsuka Pharmaceutical Factory, Inc.) to a concentration of 1 mg/mL.

Mice in the compound A administration group were given compound A by oral administration at a dose of 25 mg/kg (10 mL/kg), once per day for 7 days. Mice in the anti-PD-1 antibody administration group were intraperitoneally administered a dose of 200 μg/mouse (200 μL/mouse) on day 0 and day 3, where the starting day of administration was defined as day 0. The mice in the compound A and anti-PD-1 antibody combination group were administered both drugs in the same manner described above.

With the starting day of administration defined as day 0, the tumor volume of each mouse was measured on day 3 and day 7. The mean tumor volumes in each group are shown in Table 2 and Fig. 2.

The tumor volumes in the control group and in each group on day 7 were compared in Dunnett's multiple comparison test. The final significance level was as follows.

Control group and combination group comparison: p=0.0101

No statistically significant difference was found between the control group and the compound A treatment group, or between the control group and the anti-PD-1 antibody treatment group.

A markedly superior antitumor effect was seen in the compound A and anti-PD-1 antibody combination group compared to the control group.

[Table 2]

| | Day 0 | Day 3 | Day 7 |
|---|---|---|---|
| Control group ($mm^3$) | 61.6 | 91.1 | 132.8 |

(continued)

| | Day 0 | Day 3 | Day 7 |
|---|---|---|---|
| Compound A administered group ($mm^3$) | 58.8 | 72.5 | 107.5 |
| Anti-mouse PD-1 antibody administered group ($mm^3$) | 58.7 | 68.8 | 96.6 |
| Compound A and anti-mouse PD-1 antibody combination administered group ($mm^3$) | 58.9 | 68.6 | 86.0 |

Example 3 Antitumor effect by combination of compound A and anti-PD-1 antibody in mouse hepatocellular carcinoma cell line (BNL 1 ME A.7R.1) graft model

The antitumor effect of administering compound A and anti-PD-1 antibody was evaluated in BALB/c mice (BALB/cAnNCrlCrlj, female, Japan Charles River) with 10 mice per group.

<Establishing mouse hepatocellular carcinoma cell line BNL 1 ME A.7R.1>

The grafted cells were pre-conditioned in BALB/c mice. A suspension of the mouse hepatocellular carcinoma cell line BNL 1 ME A.7R.1 (ATCC) was subcutaneously grafted onto the right side of BALB/c mice (BALB/cAnNCrlCrlj, male, Japan Charles River), at $5 \times 10^6$ cells per mouse. After grafting, the formed tumors were extracted and finely cut. The mixture was then stirred with a GentleMACS (Miltenyi Biotec K.K.) to obtain a monocellular suspension. After passing through a cell strainer, the cells were collected by centrifugation and cultured.

The mouse hepatocellular carcinoma cell line BNL 1 ME A.7R.1 established by this method was suspended in HBSS to a concentration of $1 \times 10^8$/mL. The mixture was subsequently grafted onto the right side of BALB/c (BALB/cAn-NCrlCrlj, female, Japan Charles River) in amounts of 0.1 mL each, and the antitumor effect was evaluated.

On the 4th day after grafting, the long and short diameters of the tumors were measured using an electronic digital caliper (Digimatic™ caliper by Mitsutoyo Corp.). The mice were divided into groups in such a manner that the average tumor volumes were the same in each group. The tumor volumes were calculated by the following formula.

$$\text{Tumor volume (mm)} = \text{Long diameter (mm)} \times \text{short diameter (mm)} \times \text{short diameter (mm)}/2$$

Compound A was dissolved in water for injection (Otsuka Pharmaceutical Factory, Inc.) to a concentration of 2.5 mg/mL. The anti-mouse PD-1 antibody (BioXcell) was diluted with physiological saline (Otsuka Pharmaceutical Factory, Inc.) to a concentration of 1 mg/mL.

Mice in the compound A administration group were given compound A by oral administration at a dose of 25 mg/kg (10 mL/kg), once per day for 7 days. Mice in the anti-PD-1 antibody administration group were intraperitoneally administered a dose of 200 μg/mouse (200 μL/mouse) on day 0 and day 3, where the starting day of administration was defined as day 0. The mice in the compound A and anti-PD-1 antibody combination group were administered both drugs in the same manner described above.

With the starting day of administration defined as day 0, the tumor volume of each mouse was measured on day 3 and day 7. The mean tumor volumes in each group are shown in Table 3 and Fig. 3.

The tumor volumes in the control group and in each group on day 7 were compared in Dunnett's multiple comparison test. The final significance level was as follows.
Control group and combination group comparison: p=0.0024
No statistically significant difference was found between the control group and the compound A treatment group, or between the control group and the anti-PD-1 antibody treatment group.

A markedly superior antitumor effect was seen in the compound A and anti-PD-1 antibody combination group compared to the control group.

[Table 3]

| | Day 0 | Day 3 | Day 7 |
|---|---|---|---|
| Control group ($mm^3$) | 108.9 | 246.7 | 264.4 |

(continued)

| | Day 0 | Day 3 | Day 7 |
|---|---|---|---|
| Compound A administered group ($mm^3$) | 109.0 | 223.6 | 221.4 |
| Anti-mouse PD-1 antibody administered group ($mm^3$) | 109.0 | 238.9 | 262.5 |
| Compound A and anti-mouse PD-1 antibody combination administered group ($mm^3$) | 109.0 | 192.3 | 147.0 |

## Claims

**1.** A pharmaceutical composition for treating a tumor, comprising 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by formula (I) or its pharmaceutically acceptable salt, which is to be administered in combination with a PD-1 antagonist.

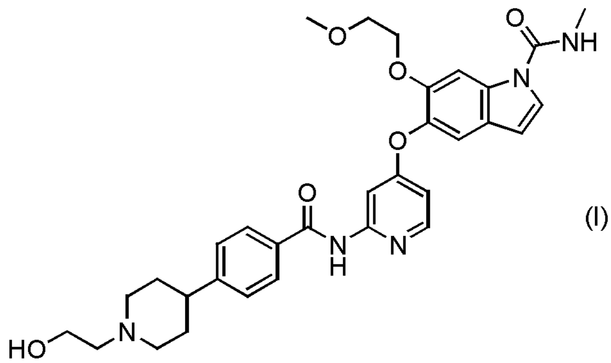

(I)

**2.** A pharmaceutical composition for treating a tumor, comprising a PD-1 antagonist, which is to be administered in combination with 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by formula (I) or its pharmaceutically acceptable salt.

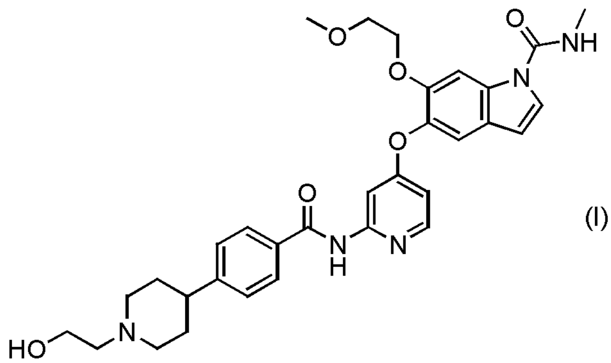

(I)

**3.** The pharmaceutical composition according to claim 1 or 2, wherein the 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by formula (I) or its pharmaceutically acceptable salt and the PD-1 antagonist are each administered simultaneously, separately, continuously or at a time difference.

**4.** The pharmaceutical composition according to any one of claims 1 to 3, wherein the pharmaceutically acceptable salt of 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by formula (I) is a 1.5 succinate.

**5.** The pharmaceutical composition according to claim 4, wherein the 1.5 succinate of 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by formula (I) is administered at 1 mg to 500 mg per day.

6. The pharmaceutical composition according to any one of claims 1 to 5, wherein the PD-1 antagonist is an anti-PD-1 antibody.

7. The pharmaceutical composition according to claim 6, wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

8. The pharmaceutical composition according to any one of claims 1 to 7, wherein the tumor is breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin's lymphoma.

9. The pharmaceutical composition according to any one of claims 1 to 7, wherein the tumor is breast cancer, stomach cancer, non-small-cell lung cancer, bladder cancer, endometrial cancer, hepatocellular carcinoma or bile duct cancer.

10. The pharmaceutical composition according to any one of claims 1 to 7, wherein the tumor is breast cancer.

11. The pharmaceutical composition according to any one of claims 8 to 10, wherein the breast cancer is locally advanced breast cancer, metastatic breast cancer or recurrent breast cancer.

12. The pharmaceutical composition according to any one of claims 8 to 11, wherein the breast cancer expresses fibroblast growth factor receptor (FGFR).

13. The pharmaceutical composition according to claim 12, wherein the FGFR is FGFR1, FGFR2 or FGFR3.

*Fig.1*

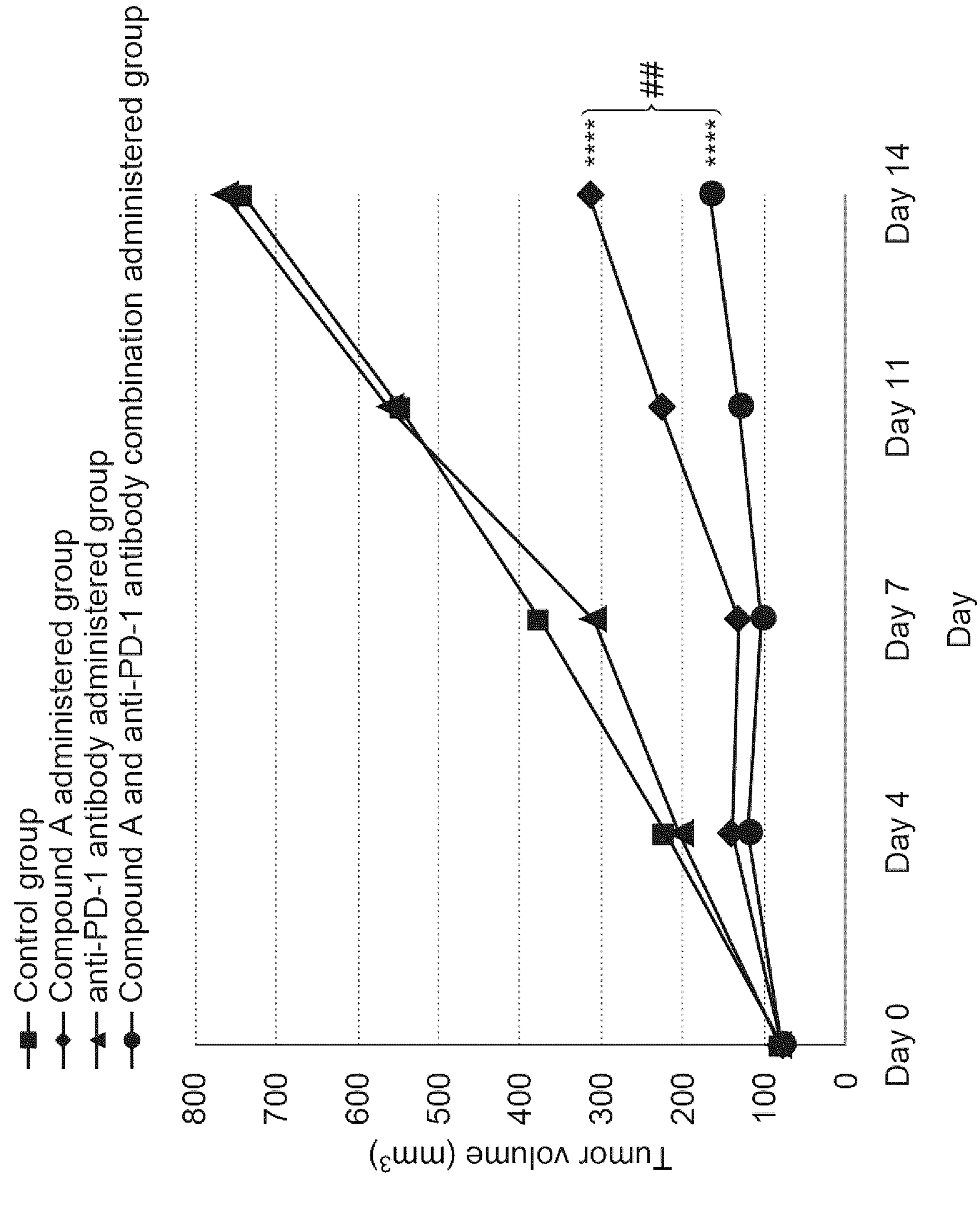
Control group
Compound A administered group
anti-PD-1 antibody administered group
Compound A and anti-PD-1 antibody combination administered group
Tumor volume (mm3)
0
100
200
300
400
500
600
700
800
Day 0
Day 4
Day 7
Day 11
Day 14
Day
****
****
##

*Fig.2*

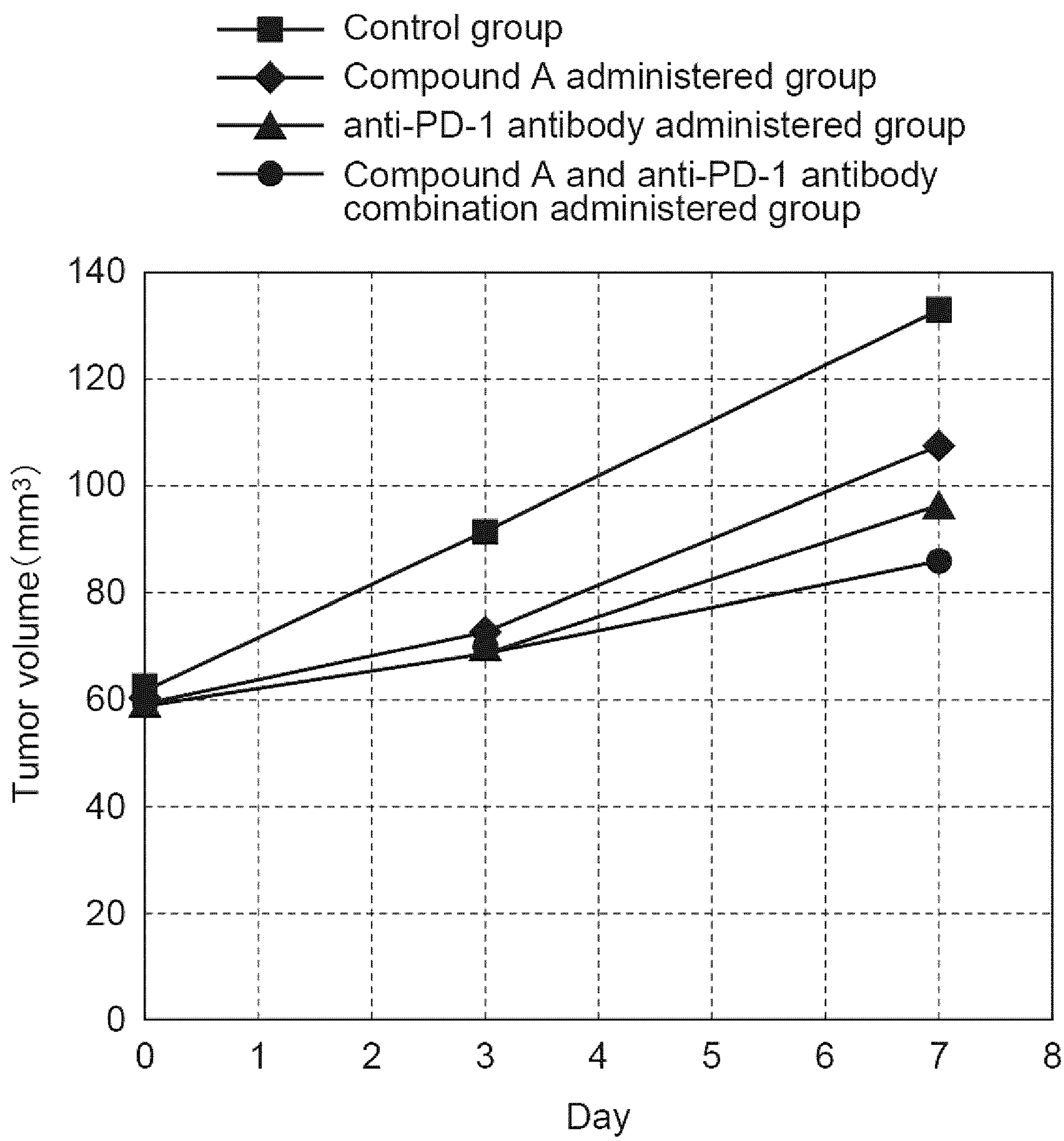
Control group
Compound A administered group
anti-PD-1 antibody administered group
Compound A and anti-PD-1 antibody combination administered group
Tumor volume (mm3)
140
120
100
80
60
40
20
0
0
1
2
3
4
5
6
7
8
Day

*Fig.3*

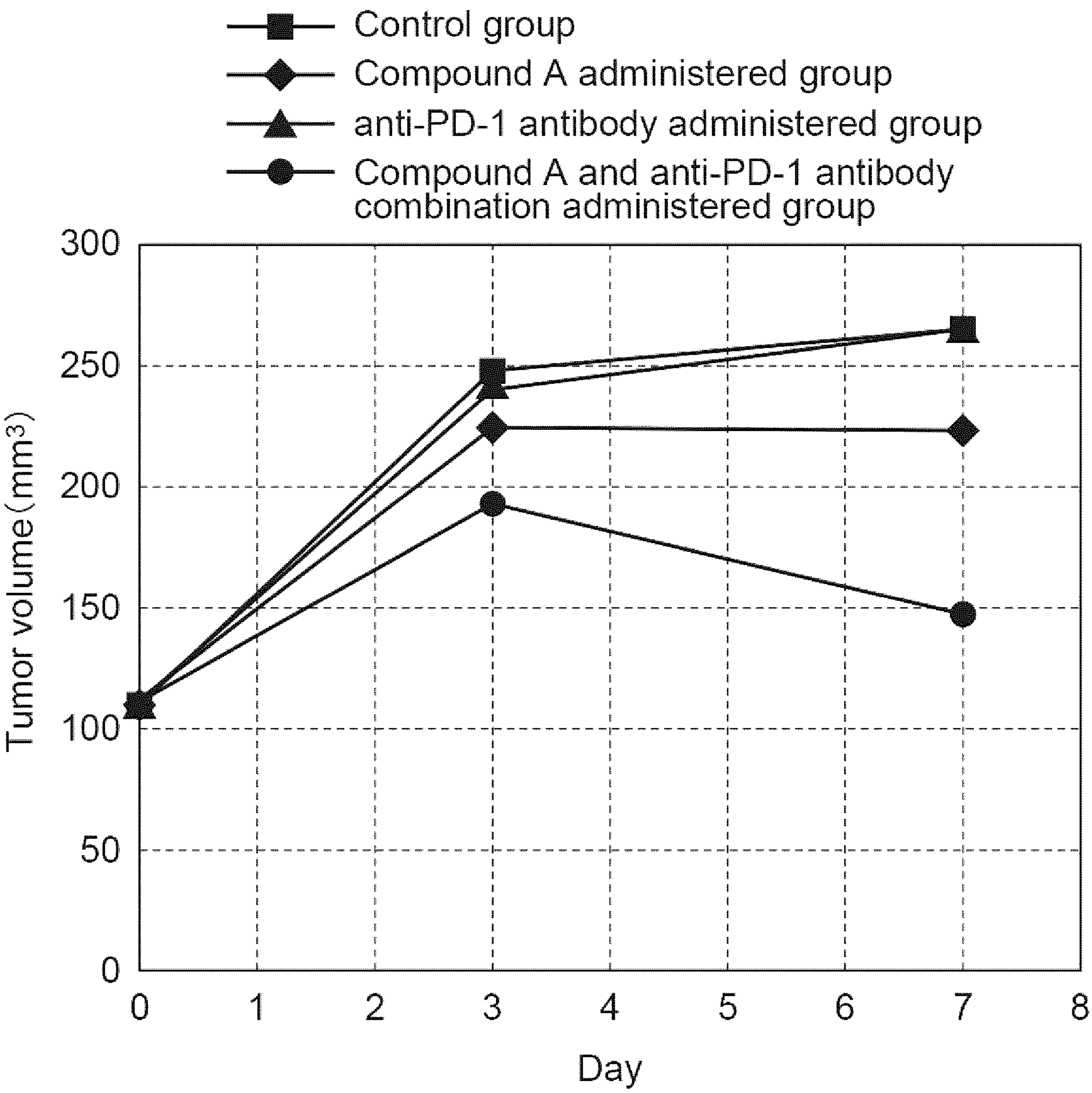
Control group
Compound A administered group
anti-PD-1 antibody administered group
Compound A and anti-PD-1 antibody combination administered group
Tumor volume (mm3)
300
250
200
150
100
50
0
0
1
2
3
4
5
6
7
8
Day

## INTERNATIONAL SEARCH REPORT

International application No.
**PCT/JP2021/039490**

**A. CLASSIFICATION OF SUBJECT MATTER**

***A61K 31/4545***(2006.01)i; ***A61K 39/395***(2006.01)i; ***A61K 45/00***(2006.01)i; ***A61P 15/00***(2006.01)i; ***A61P 35/00***(2006.01)i; ***A61P 35/04***(2006.01)i; ***A61P 43/00***(2006.01)i
FI: A61K31/4545; A61K45/00; A61P43/00 121; A61P35/00; A61K39/395 N; A61P15/00; A61P35/04; A61P43/00 111

According to International Patent Classification (IPC) or to both national classification and IPC

**B. FIELDS SEARCHED**

Minimum documentation searched (classification system followed by classification symbols)

A61K31/4545; A61K39/395; A61K45/00; A61P15/00; A61P35/00; A61P35/04; A61P43/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2017/104739 A1 (EISAI R&D MAN CO LTD) 22 June 2017 (2017-06-22) claims, paragraphs [0001], [0009], [0017], [0021], example 1 | 1-13 |
| Y | WO 2019/189241 A1 (EISAI R&D MAN CO LTD) 03 October 2019 (2019-10-03) claims, paragraphs [0001], [0017], examples 3-4 | 1-13 |
| Y | JP 2018-512391 A (MERCK SHARP & DOHME CORP.) 17 May 2018 (2018-05-17) claims, paragraphs [0005], [0013], examples 1-2 | 1-13 |
| Y | JP 2018-507220 A (BIOCLIN THERAPEUTICS, INC) 15 March 2018 (2018-03-15) claims, examples 1, 3 | 1-13 |
| Y | JP 2018-538274 A (FIVE PRIME THERAPEUTICS, INC) 27 December 2018 (2018-12-27) claims, example 3 | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 November 2021** | **14 December 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/039490**

| Patent document cited in search report | Publication date (day/month/year) | Patent family member(s) | Publication date (day/month/year) |
|---|---|---|---|
| WO 2017/104739 A1 | 22 June 2017 | US 2018/0303817 A1<br>claims, paragraphs [0001], [0010], [0019], [0023], example 1<br>EP 3391885 A1<br>CN 108367000 A<br>KR 10-2018-0094862 A | |
| WO 2019/189241 A1 | 03 October 2019 | US 2020/0375970 A1<br>claims, paragraphs [0001], [0032], examples 3-4<br>EP 3777860 A1 | |
| JP 2018-512391 A | 17 May 2018 | WO 2016/140717 A1<br>claims, paragraphs [0007], [0015], examples 1-2<br>US 2018/0092901 A1<br>KR 10-2017-0122809 A | |
| JP 2018-507220 A | 15 March 2018 | WO 2016/134234 A1<br>claims, examples 1, 3<br>US 2016/0243228 A1<br>CN 107635583 A<br>KR 10-2017-0137717 A | |
| JP 2018-538274 A | 27 December 2018 | WO 2017/091577 A1<br>claims, example 3<br>US 2017/0145102 A1<br>CN 108368174 A<br>KR 10-2018-0081606 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014235614
- US 20180015079
- US 20180303817
- WO 2019189241 A
- US 20170217935
- US 7488802 B
- US 7521051 B
- US 8008449 B
- US 8354509 B
- US 8168757 B
- WO 2004004771 A
- WO 2004072286 A
- WO 2004056875 A
- US 20110271358


### Non-patent literature cited in the description


- *The Journal of Clinical Investigation,* September 2015, vol. 125 (9
- Japanese Pharmacopoeia